(19) Europäisches Patentamt

European Patent Office

Office européen des brevets

(11) **EP 1 133 566 B1**

(12) **EUROPEAN PATENT SPECIFICATION**

(45) Date of publication and mention
of the grant of the patent:
**12.07.2006 Bulletin 2006/28**

(21) Application number: **99956249.9**

(22) Date of filing: **29.11.1999**

(51) Int Cl.:
***C12N 15/62*** (2006.01)

(86) International application number:
**PCT/GB1999/003979**

(87) International publication number:
**WO 2000/032795 (08.06.2000 Gazette 2000/23)**

(54) **DNA ENCODING FUSION PROTEINS WHICH ARE SPECIFICALLY CLEAVABLE BY COPPER (II) IONS**

DNS, DIE FUSIONSPROTEINE KODIEREN WELCHE DURCH KUPFER (II) IONEN SPEZIFISCH GESCHNITTEN WERDEN KÖNNEN

PROTEINES DE FUSION SPECIFIQUEMENT CLIVABLES PAR DES IONS DE CUIVRE (II) CODANT POUR UN ADN

(84) Designated Contracting States:
**AT BE CH CY DE DK ES FI FR GB GR IE IT LI LU MC NL PT SE**

(30) Priority: **27.11.1998 GB 9826112**

(43) Date of publication of application:
**19.09.2001 Bulletin 2001/38**

(73) Proprietor: **CELLTECH THERAPEUTICS LIMITED Slough,**
**Berkshire SL1 3WE (GB)**

(72) Inventor: **HUMPHREYS, David Paul Maidenhead,**
**Berkshire SL6 2TN (GB)**

(74) Representative: **Mercer, Christopher Paul et al**
**Carpmaels & Ransford**
**43, Bloomsbury Square**
**London WC1A 2RA (GB)**

(56) References cited:
- SMITH M A ET AL: "SPECIFIC CLEAVAGE OF IMMUNOGLOBULIN G BY COPPER IONS" INTERNATIONAL JOURNAL OF PEPTIDE AND PROTEIN RESEARCH,DK,MUNKSGAARD, COPENHAGEN, vol. 48, no. 1, 1 July 1996 (1996-07-01), pages 48-55, XP000594105 ISSN: 0367-8377
- ALLEN G ET AL: "SPECIFIC CLEAVAGE OF HISTIDINE-CONTAINING PEPTIDES BY COPPER (II)" INTERNATIONAL JOURNAL OF PEPTIDE AND PROTEIN RESEARCH,DK,MUNKSGAARD, COPENHAGEN, vol. 48, no. 3, 1 September 1996 (1996-09-01), pages 265-273, XP000623834 ISSN: 0367-8377
- NAGAI K ET AL: "GENERATION OF -GLOBIN BY SEQUENCE-SPECIFIC PROTEOLYSIS OF A HYBRID PROTEIN PRODUCED IN ESCHERICHIA COLI" NATURE,GB,MACMILLAN JOURNALS LTD. LONDON, vol. 309, 28 June 1984 (1984-06-28), pages 810-812, XP000049020 ISSN: 0028-0836
- CARTER P ET AL: "ENGINEERING ENZYME SPECIFICITY BY ''SUBSTRATE-ASSISTED CATALYSIS''" SCIENCE,US,AMERICAN ASSOCIATION FOR THE ADVANCEMENT OF SCIENCE, vol. 237, no. 4813, 24 July 1987 (1987-07-24), pages 394-399, XP000561258 ISSN: 0036-8075
- HUMPHREYS DAVID P ET AL: "Efficient site specific removal of a C-terminal FLAG fusion from a Fab' using copper(II) ion catalysed protein cleavage." PROTEIN ENGINEERING FEB., 1999, vol. 12, no. 2, February 1999 (1999-02), pages 179-184, XP000882102 ISSN: 0269-2139

Remarks:
The file contains technical information submitted after the application was filed and not included in this specification

**Description**

This invention relates to recombinant fusion proteins and to methods of cleaving such fusion proteins to yield a desired protein in native form.

In order to aid intra-cellular solubility, purification and detection, many proteins of biotechnological interest are expressed as fusion proteins including: galactosidase, IgG binding peptide, glutathione S-transferase, maltose binding protein, His-tag, myc-tag, cellulose binding domain, calmodulin binding peptide, FLAG-tag, and Strep-tag [Casadaban MJ *et al,* (1983); Nilsson B *et al,* (1987); Smith DB and Johnson KS, (1988); di Guan C *et al,* (1988); Hochuli E *et al,* (1988); Dreher ML *et al,* (1991); Ong E *et al,* 1991; Stofko-Hahn RE *et al,* (1992); Brizzard BL *et al,* (1994); and Schmidt TGM and Skerra A, (1994). For full details of the publications referred to herein, see the secion of the specification headed "REFERENCES"].

It is often undesirable for the fusion partner to remain associated with the protein of interest after purification since it may interefere with protein function, structural analysis, or be immunogenic if the protein is administered *in vivo*. Peptide sites cleaved by specific proteases such as Factor Xa, TEV, Genenase I, thrombin, enterokinase and 3C can be introduced between the two protein domains to enable cleavage of the two domains [Nagai K and Thøgersen HC, (1984); Dougherty WG *et al,* (1988); Carter P and Wells JA, (1987); McKenzie KR *et al,* (1991); LaVallie ER *et al,* (1993); Walker PA *et al,* (1994)]. However, these enzymes can be expensive, have variable effectiveness due to differences in the sequences surrounding the target site and accessibility of the site to a large enzyme, and require a further purification step to remove the proteolytic enzyme from the sample.

The upper hinge tetrapeptide sequence $^{N}DKTH^{C}$ was identified as the site of cleavage of a highly purified recombinant humanised $\gamma1$ antibody by traces of $Cu^{2+}$ [Smith M A *et al,* (1996)]. The sequence was cleaved between the Lys and Thr residues. The degree of cleavage increased with increasing temperature, length of time, and pH of incubation, and also with increasing amounts of available $Cu^{2+}$ ions. The $^{N}DKTH^{C}$ sequence was also cleaved by $Cu^{2+}$ when present in a small peptide [Smith MA *et al,* (1996)].

We have now devised an improved cleavage linker for use in the construction of recombinant DNA sequences which code for fusion proteins. The linker codes for a copper(II) site comprising a sequence of four different amino acids.

Thus according to one aspect of the invention we provide DNA coding for a fusion protein comprising two polypeptides joined by a cleavage site which does not naturally join the polypeptides and which is specifically cleavable by copper(II) ions.

For the avoidance of doubt, the term "which does not naturally join" is used herein to exclude proteins known in nature to include an amino acid sequence which is cleavable by copper(II) ions, for example as described hereinafter.

The invention also includes a vector comprising DNA coding for a fusion protein comprising two polypeptides joined by a cleavage site which does not naturally join the polypeptides and which is specifically cleavable by copper(II) ions, and host cells transformed with such a vector. The vector may be in particular an expression vector as more particularly described hereinafter. Host cells include any procaryotic or enuaryotic cells, especially bacterial, yeast, animal, e.g. mammalian, insect and plant cells.

Furthermore the invention includes a process for the production of a desired protein in native form, comprising 1) expressing the desired protein as a fusion protein in a host cell transformed with a vector according to the invention in which the desired protein is expressed as one of two polypeptides joined by a site specifically cleavable by copper(II) ions, 2) cleaving the fusion protein with copper(II) ions and 3) recovering the desired cleaved protein in native form. The fusion protein produced in this process is novel and forms a further aspect of the invention.

In all the aspects of the invention described herein the cleavage site may be any tetrapeptide which may be cleaved by copper(II) ions and includes in particular the tetrapeptide $^{N}DKTH^{C}$ as described herein and functional equivalents thereof, for example where one or more functionally equivalent amino acids is included as alternatives to those shown. Suitable substitutions include those amino acids which are generally recognisd as conservative changes for D, K, T and H, for example E for D,R for K, S for T and Y for H. Single, double or other multiple substitutions may be made. Examples of partiuclarly useful alternatives to the $^{N}DKTH^{C}$ site include $^{N}DRSH^{C}$, $^{N}EKSH^{C}$ and especially $^{N}DKSH^{C}$. DNA coding for the peptide $^{N}DKTH^{C}$ includes that described in the experimental section hereinafter and functional equivalents thereof, for example due to the degeneracy of the genetic code. DNA coding for any of the above functional equivalents of $^{N}DKTH^{C}$ is similarly readily available.

All aspects of the invention are widely applicable to the production of any desired protein, and the various terms "polypeptide", "fusion protein" and "protein" as used herein are to be understood to include any protein, polypeptide, peptide and fragments thereof from any procaryotic, eucaryotic or synthetic source, except where indicated otherwise. Particular examples include mammalian polypeptides and proteins, including enzymes; serum proteins, especially antibodies; cytokines; hormones; growth factors; receptors; structural proteins; and precursors and fragments thereof.

The fusion proteins according to the invention may be produced using standard recombinant DNA techniques involving the expression of the proteins by a host cell. Isolated DNA coding for each protein may be introduced for example into any suitable expression vector by operatively linking the DNA to any necessary expression control elements therein and transforming any suitable procaryotic or eucaryotic host cell with the vector using well known procedures, for example as described hereinafter in the experimental section. For creation of the DNA coding for the fusion protein, appropriate DNA coding for the protein of interest and its fusion partner may be fused via an oligonucleotide sequence coding for the copper cleavage site using conventional annealing procedures to ensure that the correct reading frame is maintained in the vector. Where other potential copper cleavage sites are identified in the starting DNA, these can be removed using standard mutagenesis techniques for example as described in the Examples hereinafter.

The expresssion of the fusion protein during the culture of the host cell may be monitored using standard assay procedures and at any appropriate time during or after the culture the protein may be isolated from the host cells and/or culture medium using conventional protein purification techniques such as chromatography and filtration.

Once obtained, the fusion proteins according to the invention may be cleaved using copper(II) ions to yield the desired recombinant protein. The cleavage reaction may be performed using any source of copper(II) ions, for example a copper(II) salt, e.g. $CuCl_2$, in a suitable solvent, for example an aqueous buffered solvent, at an appropriate pH and temperature. The precise reaction conditions, including the quantity of copper used, will depend on the nature and quantity of the fusion protein and the cleavage products and can advantageously be determined empirically for each protein, for example as described in the experimental section hereinafter. When an aqueous buffer is used, the buffer may advantageously be a Tris buffer. The production of the desired protein may be monitored during the cleavage reaction using any appropriate assay, based for example on the activity, immunoreactivity and/or size of the protein. The desired protein may be recovered after the cleavage reaction using standard protein isolation and purification techniques, for example as just described.

The following Figures are referred to in the Examples below. In the Figures:

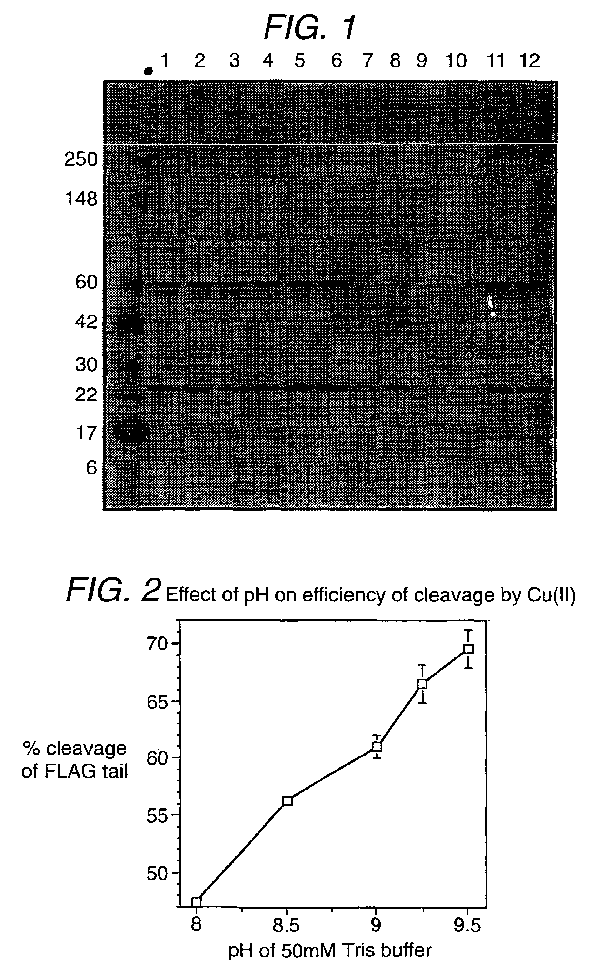

**Figure 1** Effect of buffer type on efficiency of cleavage of 'Null 2 FLAG' $F(ab')_2$ by $Cu^{2+}$

Coomassie stained non-reducing SDS-PAGE using 4-20% Tris-glycine gels. All lanes contain 1µg protein equivalents from cleavage reactions using 50mM of buffer at pH8.5 (except MES which was pH5.5) for 16h at 62°C. The size and migration of molecular weight markers are shown on the left in kDa. The cleavage reactions in lanes 1 and 2 were Tris buffered with 1 mM and 0.1 mM $Cu^{2+}$ respectively;
Lanes 3 and 4 were Bicine buffered with 1mM and 0.1mM $Cu^{2+}$ respectively;
Lanes 5 and 6 were Tricine buffered with 1mM and 0.1mM $Cu^{2+}$ respectively;
Lanes 7 and 8 were CHES buffered with 1mM and 0.1mM $Cu^{2+}$ respectively;
Lanes 9 and 10 were HEPES buffered with 1mM and 0.1mM $Cu^{2+}$ respectively;
Lanes 11 and 12 were MES buffered with 1mM and 0.1mM $Cu^{2+}$ respectively.

**Figure 2** Effect of pH on efficiency of cleavage by Cu(II)

**Figure 3** Demonstration of cleavage of FLAG tail and effect of [$Cu^{2+}$]

Non-reducing SDS-PAGEusing 4-20% Tris-glycine gels. All lanes contain 1 µg protein equivalents (unless indicated) from cleavage reactions using 50mM of buffer at pH9.0 (except MES which was pH5.5) for 24h at 62°C. Lanes 1 to 8 are Coomassie stained. The size and migration of molecular weight markers are shown on the left in kDa. The cleavage reactions in lanes 1 to 5 were Tris buffered with 5mM, 2.5mM, 1mM, 0.1 mM and 10µM $Cu^{2+}$ respectively; lane 6 was MES buffered with 1mM $Cu^{2+}$; lane 7 was Tris buffered with 10mM EDTA; lane 8 was Tris buffered with 2.5mM $Cu^{2+}$ and Complete™ protease inhibitor. Lanes 9 to 11 show immunodetection of long equivalent protein loadings with an anti-FLAG monoclonal antibody. They are all Tris buffered and contain 2.5mM $Cu^{2+}$ 1 mM $Cu^{2+}$, and 10mM EDTA respectively. Lane 12 contains a 1µg protein loading silver stained gel of a Tris buffered cleavage reaction containing 1mM $Cu^{2+}$.

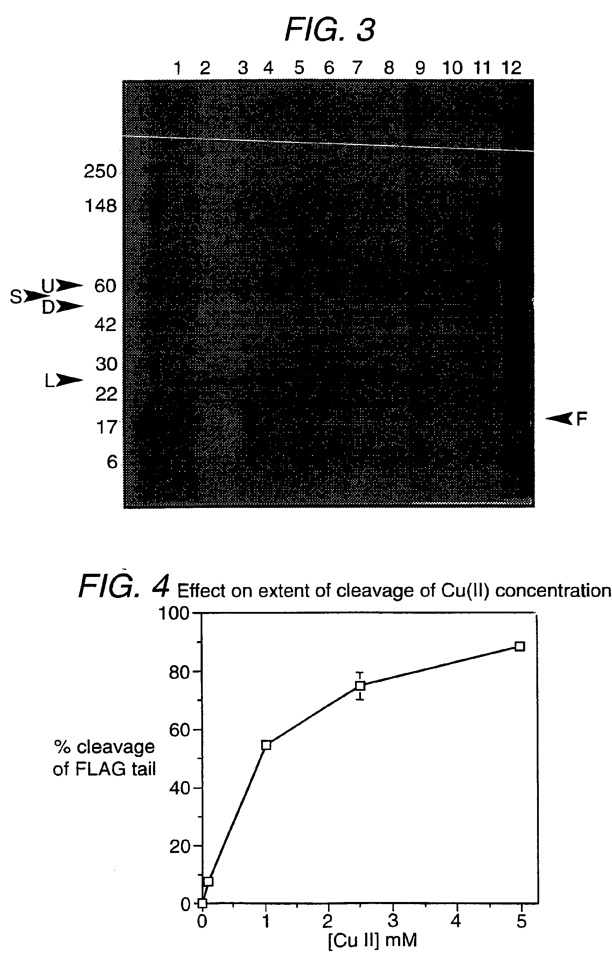

**Figure 4** Effect on extent of cleavage of Cu(II) concentration.

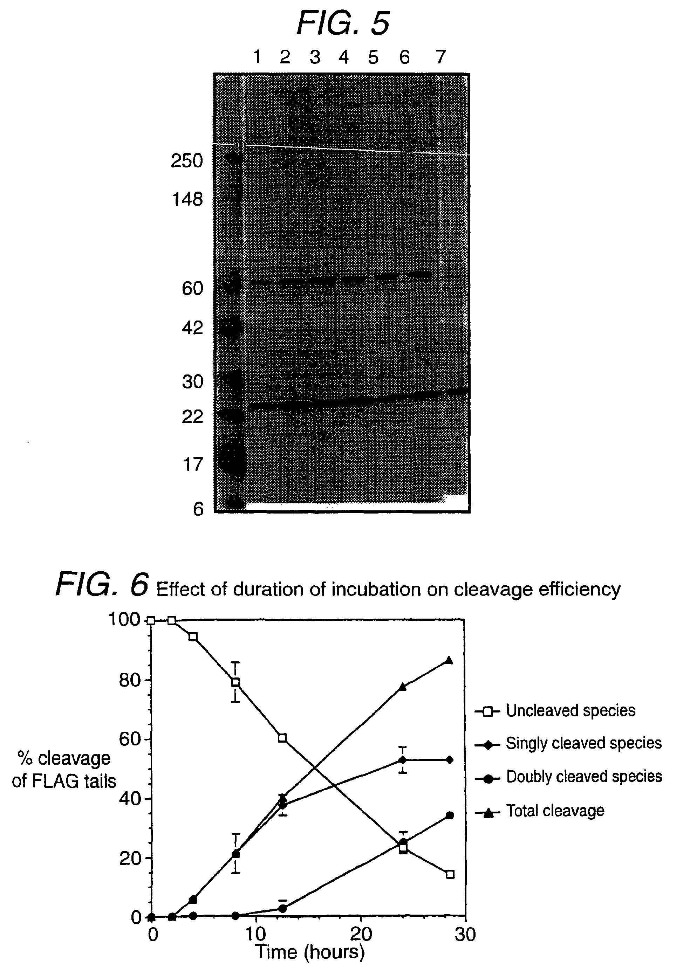

**Figure 5** Effect of metal ion on cleavage efficiency.

Coomassie stained non-reducing SDS-PAGE using 4-20% Tris-glycine gels. The size and migration of molecular weight markers are shown on the left in kDa. All lanes contain 1µg protein equivalents from cleavage reactions using 50mM of Tris buffer at pH9.0 for 24h at 62°C, and contain 1mM $Cu^{2+}$, $Fe^{3+}$, $Mg^{2+}$, $Mn^{2+}$, $Ni^{2+}$, $Zn^{2+}$ and 2.5mM $Cu^{2+}$ in lanes 1 to 7 respectively.

**Figure 6** Effect of duration of incubation on cleavage efficiency.

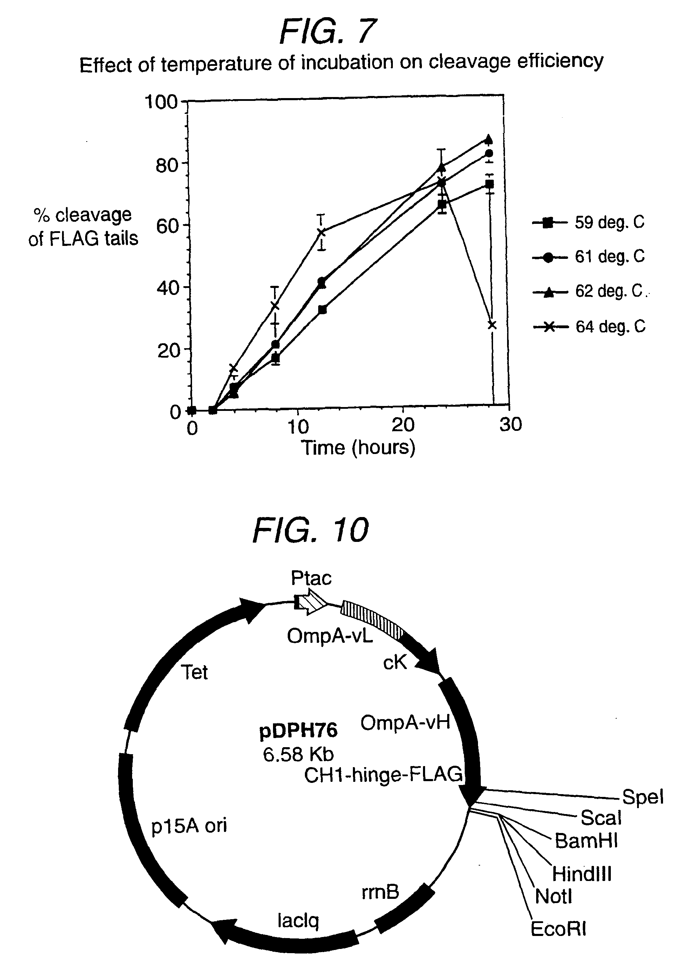

**Figure 7** Effect of temperature of incubation on cleavage efficiency.

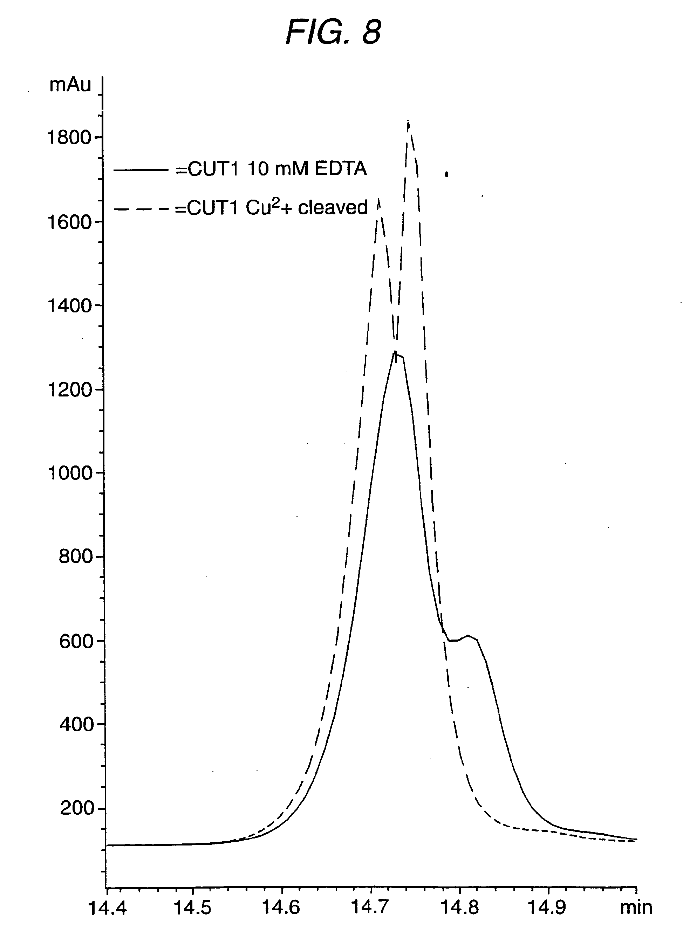

**Figure 8** HPLC mobility of cleaved and uncleaved CUT1 peptide.

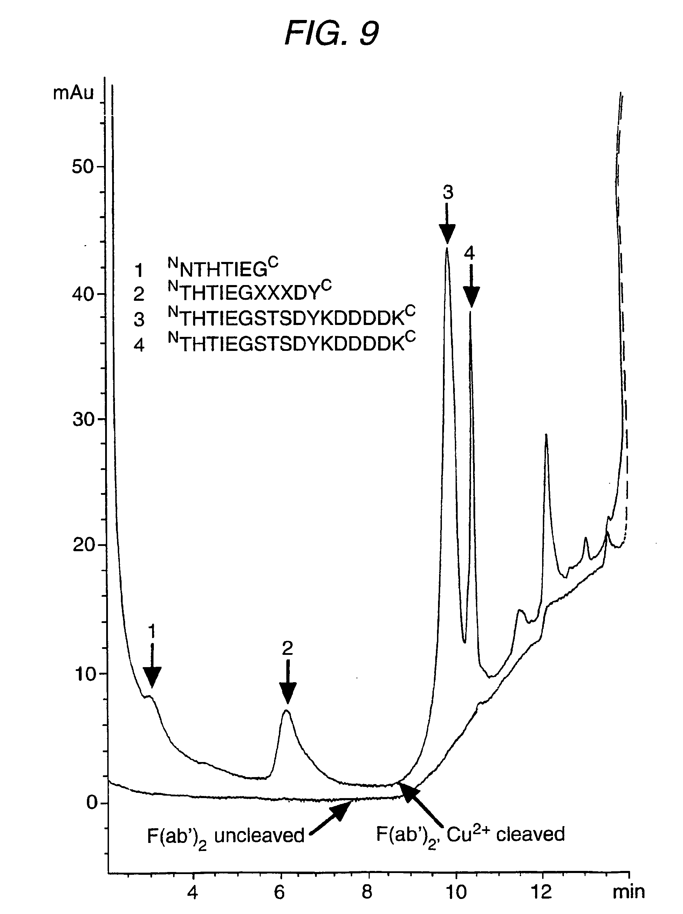

**Figure 9** HPLC mobility of peptides collected and seq.uenced after cleavage of Null 2 FLAG F(ab')$_2$

**Figure 10** Plasmid mgp for pDPH76.

## EXAMPLES

The following sections describe aspects of the invention in more detail. The following abbreviations are used: Fab' - antigen binding antibody fragment; F(ab')$_2$ - dimeric Fab'; LC - light chain; HC - heavy chain.

In the following, the use of Cu$^{2+}$ as an alternative to enzymatic cleavage of fusion proteins is demonstrated using a humanised γ1 Fab' with a C-terminal FLAG peptide as a model protein. A $^N$DKTH$^C$ site was introduced between the hinge and the FLAG peptide after mutagenesis of the native $^N$DKTH$^C$ sequence in the upper hinge to create a "null" site no longer cleaved by Cu$^{2+}$.

## Experimental protocol

### Construction of 'Null 2 FLAG' Expression Plasmid

Plasmid pDPH76 encoding 'Fab 40.4 hinge 1 Δ inter Null 2 FLAG' was derived from plasmid pDPH40 which encodes 'Fab 40.4 hinge 1/2 Δ inter'. pDPH40 has a Spe 1 restriction site introduced at the 3' end of the HC cistron - where the interchain disulphide Cys had been mutated to a Ser, along with an adjacent Ser to Thr change (Humphreys DP *et al*, 1997). This enabled cloning of the region encoding the hinge, Cu$^{2+}$ site, spacer and FLAG as an *Spe* I-*Eco* RI oligonucleotide cassette after annealing the oligonueleotides:
5'TAGTCTGCAGGTGCTGACCTGCCCGCCGTGTCCGGATAAAACCCA    TACCATCGAAGGCAGTACTAGCGATTATAAAGATGATGATGATAAATG  ATGAGGATCCAAGCTTGCGGCCGCG  3', and 5' AATTCGCGGCCGCA AGCTTGGATCCTCATCATTTATCATCATCATCTTTATAATCGCTAGTAC TGCCTTCGATGGTATGGGTTTTATCCGGACACGGCGGGCAGGTCAG CACCTGCAGA 3' resulting in plasmid pDPH76, (Figure 10). The newly constructed coding region also contained unique *Bsp*E I and *Sca* I restriction sites. Codons preferred by *E. coli* were chosen [Wada KN *et al,* (1991)]. The sequence of the oligonucleotide encoded region was confirmed by sequencing of both strands using PRISM cycle sequencing kit and an ABI 373A sequencing machine.

### Production and Purification of 'Null 2 FLAG' F(ab')$_2$

High density bacterial fermentations of strain W31 10 bearing pDPH76 were performed as described previously [Humphreys DP *et al* (1997)]. Extraction of periplasmic material, Protein G purification of Fab', F(ab')$_2$ production and Phenyl Sepharose purification were performed as described previously [Humphreys *et al*, (1998)]. After purification of F(ab')$_2$, the protein was concentrated and buffer exchanged with several volumes of PBS using an Amicon pressurised stirred cell with a 10kDa cut-off membrane to concentrations between 0.5 and 1.6mgml$^{-1}$, and sterilised with a 0.22μM filter for storage at 4°C.

### Cu$^{2+}$ Cleavage Reactions

F(ab')$_2$ was included in all reactions at 0.33mgml$^1$, Tris and other buffers were used at a final concentration of 50mM (from a 500mM stock solution). Buffers were made to pH with HCl, and Tris was used at pH 9.0 unless otherwise stated. All metal ions tested were chloride salts dissolved in dH$_2$O and were used from 10x stocks for each concentration tested. Reactions were made up to volume with dH$_2$O in 0.5ml eppendorf tubes, vortex mixed and centrifuged briefly. Reaction volumes were most commonly 15μl, but were also scaled up to 150μl. In order to eliminate evaporation during lengthy incubation at temperatures ranging from 59°C to 64°C reactions were covered in a layer of paraffin oil. Incubations at 62°C were in an air incubator, whilst other temperatures were in a Biometra TRIOThermoblock with heated lids. For SDS-PAGE 1μg samples were withdrawn through the paraffin oil for immediate electrophoresis or into 10mM EDTA and stored at 20°C prior to electrophoresis during time course experiments. Samples destined for HPLC analysis had paraffin oil removed by pipetting and ether extraction. EDTA free Complete™ protease inhibitor (Boehringer Mannheim, U.K.) was used as per manufacturers instructions.

**SDS-PAGE Analysis of 'Null 2 FLAG' Cleavage by Cu$^{2+}$**

1$\mu$g per lane of Cu$^{2+}$ treated samples were electrophoresed on 4-20% Tris-glycine gels (Novex, U.K.) after boiling in non-reducing SDS-PAGE loading buffer for 3min. Gels were stained for 10min with Coomassie brilliant blue, and then destained before analysis by laser scanning densitometry on a Molecular Dynamics model 300A machine using ImageQuaNT software version 4.2 or drying between cellulose membranes (Novex, U.K.). 'Null 2 FLAG' F(ab')$_2$ lacks the interchain disulphide bond and so separates into di-HC and free LC species during non-reducing SDS-PAGE. Since each F(ab')$_2$ molecule contains two Cu$^{2+}$ cleavage sites, three species are possible after incubation with Cu$^{2+}$: Uncut (U), singly cleaved (S), and doubly cleaved (D) (Figure 3). Species with FLAG tails removed migrate more rapidly than uncut species during SDS-PAGE making them easy to identify (Figure 3). The lower band representing doubly cleaved species (D) is not immunoreactive to a anti-FLAG antibody, demonstrating that both FLAG tails have been removed from this species (lanes 9 and 10, Figure 3). F(ab')$_2$ treated with 10mM EDTA shows only singly cleaved species by immuno-detection even after a 24h incubation at 62°C (lane 11, Figure 3). Cleaved FLAG tail could be visualised after silver staining of SDS-PAGE (band F lane 12, Figure 3).

Percent cumulative cleavage was calculated after measurement of the area of absorbance for each of the peaks for the three species thus: cumulative cleavage

$$\frac{(\text{absorbance (S)} + \text{absorbance (D)})}{(\text{absorbance (U)} + \text{absorbance (S)} + \text{absorbance (D)})} \times 100$$

Gels were silver stained with 'Silver Stain II' from Daiichi Pure Chemicals Co. Ltd., Tokyo, Japan. Western blotting was performed as described previously [Humphreys DP *et al*, (1997)], except that the primary antibody used was anti-FLAG monoclonal M2 at 1/500 dilution (Kodak, U.K.), which was revealed with ExtrAvidin-HRP (Sigma) at 1/500. 'Rainbow' markers were used as molecular weight standards throughout (Novex, U.K.).

**Surface Plasmon Resonance**

Kinetic analysis to determine the on and off rates for antigen binding to 'Null 2 FLAG' F(ab')$_2$ molecules was performed using a BIACORE 2000 (Biacore AB). 'Null 2 FLAG' F(ab')$_2$ molecules were captured by an anti-human IgG, which is immobilised on the sensor chip surface, followed by an injection of soluble antigen. Affinipure goat anti-human Ig, F(ab')$_2$ fragment specific (Jackson ImmunoResearch) was immobilised on a Sensor Chip CM5 via amine coupling chemistry to a level of 15500RU. HBS buffer (10mM HEPES pH7.4, 0.15M NaCl, 3mM EDTA, 0.005% Surfactant P20, Biacore AB) was used as the running buffer with a flow rate of 10$\mu$l/min. An injection of 'Null 2 FLAG' F(ab')$_2$ was captured by the immobilised anti-human IgG to a level between 340-390RU. Soluble antigen was injected over the captured 'Null 2 FLAG' F(ab')$_2$ surface at 2 and 0$\mu$g/ml. The surface was regenerated by injecting 2 x 5$\mu$l of 30mM HCl. The sensorgram for soluble antigen binding was corrected with the control buffer sensorgram. Kinetic parameters were calculated using BIA evaluation 2.1 software.

**Mass Spectrometry**

Molecular mass for Fab' and F(ab')$_2$ was determined using Fisons VG Quattro 1 triple quadrupole equipment in electrospray ionisation mode. F(ab')$_2$ samples were desalted to remove Tris by multiple volume exchanges with 10mM ammonuim acetate using Microcon concentrators with a 10kDa membrane cutoff size (Amicon, U.K.).

**Amino Acid Composition Analysis**

This was carried out at the Dept. of Mol. and Cell Biol., University of Aberdeen, U.K. on a ABI 420A machine after hydrolysis with 6M HCL for 40min at 160°C under Argon.

**HPLC purification and N-terminal sequencing of cleaved FLAG tail from F(ab')$_2$.**

HPLC purification - reaction mixtures containing cleaved/uncleaved F(ab')$_2$ were resolved by high performance liquid chromatography, using an Aquapore RP300, 7 micron, 2.1 x 100mm column on a Hewlet Packard HP1090 with on-line diode array for spectral analysis. Components were resolved with a linear gradient of 2 to 95% acetonitrile in water/triflouracetic acid (0.1% v/v), at a flow rate of 0.5 ml/min, at 21°C. Peaks absorbing at 214nm were collected and concentrated under vacuum to remove acetonitrile for peptide sequencing.

Peptide sequencing - peptides in fractions were bound to polyvinylidene difluoride membrane (Prosorb, Applied Biosystems), and 100mg polybrene (Biobrene, Applied Biosystems) applied to, and air-dried on, the membrane. Each peptide on Prosorb membrane was then analysed by sequencing on an Applied Biosystems 470A with on-line 120A analyser.

## HPLC analysis of peptide cleavage by Cu$^{2+}$

Peptides were dissolved in dH$_2$O and used at 0.5mM final concentrations in incubations with Tris pH9.0 at 50mM, and CuCl$_2$ from 2.5mM to 100$\mu$M made up to final reaction volume of 20$\mu$l with dH$_2$O in 0.5ml eppendorf tubes, vortex mixed and centrifuged briefly before overlaying with paraffin oil and incubating at 62°C for 16-24h. Samples had paraffin oil removed by pipetting and ether extraction. Peptides were analysed by HPLC, using a Symmetry C18 column (3.5 micron, 4.6 x 150mm) on a Hewlet Packard HP1090 with on-line diode array for spectral analysis. The components of the sample were resolved on a linear gradient, from 5 to 95% acetonitrile in trifluoracetic acid (0.1% v/v), at a flow rate of 0.5ml/min, at 21°C. Elution was monitored at 214nm.

## RESULTS

### Engineering of Fab' with C-terminal Cu$^{2+}$ cleavage site.

The Fab' was derived from a previously described humanised $\gamma$l Fab' ('Fab 40.4 hinge 1 $\Delta$ inter') [Humphreys DP $et$ $al$, (1997)] This binds a human cytokine with high affinity and is purified in high yield from the periplasm of $E.coli$ after high ceii density fermentation of strains bearing Fab' expression plasmid. The 'Fab 40.4 hinge 1 $\Delta$ inter' has two cysteines in an intact $\gamma$1 hinge and lacks the inter light chain-Fd disulphide bond (hereafter, interchain), due to mutagenesis of the interchain cysteines to serines. In order to demonstrate that the 'null' Cu$^{2+}$, site was not cleaved by Cu$^{2+}$, the Fab' was dimerised to F(ab')$_2$. Non-reducing SDS-PAGE of F(ab')$_2$ lacking the interchain bond produces a readily identifiable di-heavy chain species with a mass of ~50kDa which can remain dimeric only if the 'null' site is not cleaved by Cu$^{2+}$. The smaller size of the di-HC species compared to the F(ab')$_2$ species also facilitated accurate analysis of the extent of protein cleavage by non-reducing SDS-PAGE.

All four residues in the native $^N$DKTH$^C$ sequence of the upper hinge of 'Fab 40.4 hinge 1 $\Delta$ inter' were mutated in order to destroy the Cu$^{2+}$ cleavage site, resulting in the protein termed 'Null 2 FLAG'. Following the middle hinge there is a short hydrophilic/flexible spacer region, followed by a Cu$^{2+}$ cleavage site $^N$DKTH$^C$, and finally a FLAG peptide [Brizzard BL $et$ $al$, (1994)]. The C-terminii sequences of the two heavy chains are shown below for comparison.

'Fab 40.4 hinge 1 $\Delta$ inter' heavy chain

$^N$. .EPKTS<u>DKTH</u>TCPPCPA $^C$

Cu$^{2+}$

'Fab 40.4 hinge 1 $\Delta$ inter Null 2 FLAG' heavy chain

$^N$. .EPKTS<u>LQVL</u>TCPPC<u>DKTH</u>TIEGSTS<u>DYKDDDDK</u> $^C$

Null2          Cu$^{2+}$                    FLAG

The 'Null 2 FLAG' Fab' was found to be intact after purification from $E.coli$ cell fermentation cell pastes by Protein G chromatography by mass spectrometry. The observed masses for light and heavy chain were 23,382.66 $\pm$ 1.18 and 26,739.62 $\pm$ 1.87 respectively, compared to the predicted masses of 23,384.25 and 26,742.04 respectively. Hence, the introduced Cu$^{2+}$ site, flexible spacer, and FLAG peptide were not cleaved by $E.coli$ proteases or media components.

### Effect of buffer composition and pH.

It was known that the cleavage reaction of the native upper hinge site was favoured by alkaline pHs [Smith MA $et$ $al$, (1996)]. A strong buffer was needed to enable investigation of the optimal pH since concentrated CuCl$_2$ is acidic. Buffers with primary amines are known to have a binding capacity for Cu$^{2+}$ ions [Dawson RMC $et$ $al$, (1989)]. We tested the efficiency of cleavage reactions using 50mM Tris, Bicine, Tricine, CHES, and HEPES as buffers at pH 8.5 using $Cu^{2+}$ concentrations of lmM and IOO$\mu$M. Surprisingly, Tris resulted in the most efficient cleavage, Bicine and Tricine minimal cleavage, whilst CHES and HEPES resulted in loss of F(ab')$_2$ material (Figure 1). The $Cu^{2+}$ binding capacity of the Tris buffer (which contains 1° amines) may actually be beneficial, acting as a $Cu^{2+}$ chelation/buffer system and by presention of $Cu^{2+}$ to the Fab' $Cu^{2+}$ binding site.

Tris was then tested at pHs 8.0, 8.5, 9.0, 9.25, and 9.5 to define the optimal pH for cleavage reactions. The results in Figure 2 show clearly an increase in protein cleavage with increasing pH. pH 9.0 was chosen for all subsequent cleavage reactions as a compromise between efficient cleavage, effective buffering, and the desire to use mild incubation conditions. Incubation with 0.1mM, 1mM, and 2.5nmM $Cu^{2+}$ at pH 5.5 in MES buffer did not result in cleavage of the FLAG tail (lanes 11 and 12 Figure 1, and lane 6 Figure 3)

### Effect of Cu$^{2+}$ Concentration.

The [$Cu^{2+}$] giving optimal protein cleavage was determined empirically. Maximum protein and peptide cleavage was found previously to be at a molar ratio of between 0.5 and 1 $Cu^{2+}$:protein/peptide in a non-Tris buffer [Smith MA *et al,* (1996) and Allen G & Campbell RO(1996)]. Since Tris buffer has a significant $Cu^{2+}$ binding capacity, prediction of the effective maximum [$Cu^{2+}$] was difficult. A range of $Cu^{2+}$ concentrations from 10mM to 10$\mu$M were tested. 10mM and 7.5mM $Cu^{2+}$ were found to cause immediate and overnight precipitation respectively, but the SDS-PAGE analysis for the remainder of the titration are shown in Figure 3. Quantification of the effect of [$Cu^{2+}$] is shown in Figure 4. This shows that there is a clear dependence of the extent of cleavage upon [$Cu^{2+}$]. Such cleavage was completely inhibited by inciusion of EDTA at a final concentration of 10mM (lane 7, Figure 3). The extent of cleavage with 2.5mM $Cu^{2+}$ was unaffected by inclusion of a broad range protease inhibitor (lane 8 Figure 3), suggesting that this highly purified F(ab')$_2$ sample was not being subjected to the effects of a contaminating protease. No cleavage of 'Null 2 FLAG' is seen after 24hr incubation with $Ca^{2+}$, $Fe^{3+}$, $Mg^{2+}$, $Mn^{2+}$, $Ni^{2+}$, or ions $Zn2^2$ at 1mM (Figure 5), demonstrating that the cleavage event was due specifically to the presence of $Cu^{2+}$ ions.

### Effect of Duration of Incubation.

The dynamics of cleavage at 62°C with 2.5mm $Cu^{2+}$ are non-linear (Figure 6). There was a short lag phase of approximately 2h before significant protein cleavage was observed. Cleavage proceeded fairly linearly until approximately 12-14 hours (~50% cleavage), followed thereafter by a slowing in the accumulation of cleaved species. After an overnight incubation with 2.5mM $Cu^{2+}$ approximately 53% of $Cu^{2+}$ sites had been cleaved, 77% after a 24h incubation, and reaching 86% after 28.5h. The lag phase may be an indicator of a $Cu^{2+}$ availability effect - equilibration of $Cu^{2+}$ between Tris and F(ab')$_2$ binding sites, or generation of reactive peptide cleaving species [Allen G and Campbell RO (1996); Wolff SP *et al,* (1986); and Garrison WM (1968)]. The decrease in cleavage rate after 14h probably simply reflects the decreasing availability of uncleaved substrate.

### Effect of Temperature

The effect of temperature of incubation on the efficiency of cleavage by $Cu^{2+}$ was investigated by following a time course of cleavage at 59°C, 61 °C, and 64°C, allowing comparison with that observed at 62°C. The results (Figure 7) show that increasing the temperature increases both the initial rate of accumulation of cleaved species and the final total percent cleavage after a 28.5h incubation. However, at 64°C protein loss was observed during the incubation, resulting in a sharp decrease in cleaved species and a concomitant increase in measurement errors and loss of LC.

The particular maximum tolerated temperature in a $Cu^{2+}$ cleavage reaction will depend upon substrate protein type and concentration and [$Cu^{2+}$] buffer type and concentration. We observe a small but significant amount ($\approx$25 %) of'Null 2 FLAG' loss at 62°C toward the end of the incubation, but non after incubation at 59°C for 28.5h.

Lack of the interchain disulphide bond in 'Null 2 FLAG' may play an important role in the temperature sensitivity of this protein during prolonged incubation at elevated temperatures. The LCs and HCs of this F(ab')$_2$ can presumably part transiently in solution, and the rate of this parting will increase with temperature. Exposure of normally buried surfaces, particularly on the HC may lead to protein:protein and protein:tube aggregation events.

### Specificity of Cu$^{2+}$ Catalysed Protein Cleavage

In order to discover whether 'Null 2 FLAG' was being cleaved as expected between the Lys and Thr of the introduced [N]DKTH[C] sequence, a sample of 'Null 2 FLAG' cleaved with 2.5mm $Cu^{2+}$ for 18 hours was analysed by electospray mass spectrometry. Depending upon the extent of cleavage each di-HC species can have one or both FLAG tails removed, resulting in species S and D as seen in Figure 3 (singly and doubly cleaved respectively). Cleavage between Lys and Thr would result in species with predicted masses of 51573.35 and 49664.62 for the singly and doubly cleaved species respectively. We find that the two strongest peaks relating to di-HC had observed masses of 51565.68 $\pm$ 10.47 and 49662.08 $\pm$ 11.16, which are in agreement with the predicted masses for di-HC with one and two FLAG tails removed. In addition, we find no evidence of the species that would be generated if the di-HC were cleaved at the 'Null 2' site, nor for non-specific $Cu^{2+}$ cleavage fragments.

To confirm that the 'Null 2' sequence was not cleavable we tested the effect of incubation of the peptides 'NULL 2' ([N] Ac-VEPKTSLQVLT-NH$_2$ [c]) and 'CUT 1' ([N] Ac-VEPKTSDKTHT-NH$_2$ [c]) with $Cu^{2+}$ in 50mM Tris pH 9.0 at 62°C. The peptide 'NULL 2' was not cleaved by $Cu^{2+}$ from 0.1mM to 2.5mM (data not shown), whereas 'CUT 1' was seen to be cleaved by the higher concentration of $Cu^{2+}$ (Figure '8). There is a clear change in the mobility and distribution of the two peaks that represent CUT1 peptide after treatment with 2.5mM $Cu^{2+}$, however we cannot explain the unusual doublet migration of this peptide during HPLC. Pure CUT1 was found by mass spectrometry to be $\geq$ 95% full length material (mass 1282.5). After incubation of CUT1 with 2.5mM $Cu^{2+}$, collection of peaks from HPLC purification, and mass spectrometry show the presence of a major and a trace species with molecular masses of 944.5 and 1281.8 respectively. CUT1 peptide cleaved between the Lys and Thr of the cleavage site (Ac-VEPKTSDK-OH) has a predicted mass of 944.48, whilst the full length and unmodified CUT1 peptide has a predicted mass of 1282.5 demonstrating that the change in peaks shown for the $Cu^{2+}$ treated material in Figure 8 represent correct cleavage of CUT1 peptide.

N-terminal amino acid sequencing of HPLC purified $Cu^{2+}$ cleaved FLAG tail was performed to ascertain the integrity of the cleavage site amino acids. Fractions relating to four peptide peaks with different migration properties during HPLC purification were collected and sequenced (Figure 9). All were found to have the correct N-terminal [N]THTIEG[C] sequence confirming accurate cleavage at the predicted site, and that the Thr and His residues in the $Cu^{2+}$ site had not been destroyed or modified during the reaction. The two most abundant fractions were collected at the start of the elution gradient and gave complete sequence of [N]THTIEGSTSDYKDDDDK[C]. The reason for the heterogeneity of FLAG tail peptide migration during HPLC is not known.

## Effect of Cleavage Conditions on Protein Function

SDS-PAGE and mass spectrometry analysis showed that the cleavage conditions had not grossly affected the integrity of the 'Null 2 FLAG' protein, as witnessed by the absence of new protein fragments. We took advantage of the antigen binding ability of the Fab 40.4 derived 'Null 2 FLAG' to investigate whether more subtle changes in the protein occur after cleavage reaction incubation. Modification of residues in the CDRs might well result in a loss of antigen binding. Gly, Pro, Lys, and His in particular are known to be sites for protein damage by divalent metal ions [Dean RT *et al*, (1989); Easton CJ *et al*, (1997); Hawkins CL and Davies MJ (1997)]. The CDRs of 'Null 2 FLAG' contain a total of 5 Gly, 2 Lys, and 1 Pro residues, so we might expect that any non-specific damage to the F(ab')$_2$ might be reflected more measurably in loss of affinity due to damage specifically to the CDRs. Surface plasmon resonance affinity measurements (Table I) show that overnight incubation at pH 9.0 with or without 2.5mM $Cu^{2+}$ do not significantly affect the affinity of 'Null 2 FLAG' F(ab')$_2$. The very minor apparent loss of affinity is actually caused by the incubation procedure, and not due to the $Cu^{2+}$ itself. This may be a reflection of the ability of the LC and HC of the F(ab')$_2$ to part, due to the absence of the interchain disulphide bond.

**Table 1**

| Treatment | $k_{ass}$ (M) | $k_{ass}$ (M) | kD (M) |
|---|---|---|---|
| 50mM Tris pH 9.0, 62°C, 16h, 2.5mM $Cu^{2+}$ | $4.22 \times 10^{-5}$ | $2.08 \times 10^{-4}$ | $4.93 \times 10^{-10}$ |
| 50mM Tris pH 9.0, 62°C, 16h, 10mM EDTA | $4.27 \times 10^{-5}$ | $1.85 \times 10^{-4}$ | $4.33 \times 10^{-10}$ |
| Untreated | $3.93 \times 10^{-5}$ | $1.28 \times 10^{-4}$ | $3.26 \times 10^{-10}$ |

In addition, we performed a total amino acid composition analysis on 'Null 2 FLAG' F(ab')$_2$ that had been incubated for 16h at 62°C in 50mm Tris pH 9.0 with or without 2.5mM $Cu^{2+}$. There were no new peaks in the $Cu^{2+}$ treated sample that might be evidence of modified amino acids, and no significant difference in the amino acid composition between the two samples as evidence of degradation of particular amino acids.

## Discussion

The Results show evidence for use of the tetra-peptide sequence [N]DKTH[C] as a site for specific cleavage of fusion proteins by cupric ions. In the model protein a C-terminal FLAG peptide is cleaved off exactly between the Lys and Thr of the target sequence as shown by mass spectrometry of the remaining N-terminal F(ab')$_2$ Part, and N-terminal sequencing of liberated C-terminal FLAG peptide.

The cleavage is efficient, reaching levels of 71%, 81%, and 86% after 28.5h incubations at pH9.0, and 59°C, 61°C, 62°C respectively. The cleavage site does not need to be dimeric in order to be cleaved, since we see significant accumulation of F(ab')$_2$ species with both one and two FLAG tails removed. We find no evidence for gross deleterious effects on the structure or activity of the F(ab')$_2$ used in this system, even after incubation with 2.5mM Cu$^{2+}$ for 16h at 62°C, pH 9.0. It is clear that the high incubation temperature is the most critical element in determining the quality of protein after the cleavage treatment. With this particular protein the 5°C difference between 59°C and 64°C changes the incubation from one that gives a yield of 71 % cleavage and unmeasurable protein loss to one with ≥70 % protein loss after a 28.5h incubation. However, the maximum tolerated temperature for other proteins needs to be determined empirically.

The efficiency of cleavage reactions is high even without final optimisation of the cleavage process for' Null 2 FLAG'F(ab')$_2$. Simply by increasing the [Cu$^{2+}$] from 2.5mM to ≤5mM, and the pH to 9.5 we could increase the extent of cleavage at any given time.

The 'Null' site of'Null 2 FLAG' contains changes to all four residues ($^N$DKTH$^C$ → $^N$LQVL$^C$). To minimise the risk of immunogenicity against Fab' material cleaved by this technique requires making a minimal number of changes in the native $^N$DKTH$^C$ site whilst remaining uncleavable by Cu$^{2+}$. The same four residues (D, K, T, and H) are involved in the metal binding site of serum albumins, and so are clearly capable of chelating divalent cations [Sadler PJ et al, (994)]. However, in the creation of a 'Null' site the relative effect of each residue upon the ability of the site to be cleaved, rather than simply chelate Cu$^{2+}$ is critical. The pH dependence of the cleavage reaction and peptide variants implicate the importance of the His residue in the cleavage reaction [Allen Gand Campbell RO, (1996)]. It is possible that a single mutation of this residue, or combined with mutation of the Thr residue to a small or hydrophobic residue might result in this remaining uncleavable by Cu$^{2+}$.

No evidence was found for cleavage of Null 2 FLAG' F(ab')$_2$ at sites other than that of the $^N$DKTH$^C$ sequence. This is consistent with the absence of this tetrapeptide from the 1° structure of 'Fab 40.4'. In fact we find that the NDKTH$^C$ sequence is relatively rare. Only 167 matches are found against 256,226 entries searched in the NCBI protein database. To be cleaved by Cu$^{2+}$ this sequence must be exposed, and probably also requires a flexible/disordered structure. This combination of rarity of both 1° and 3° structure of the Cu$^{2+}$ cleavage site makes the target specific enough to be a general protein cleavage site. The protein retains only two amino acids of the site after cleavage, and so these are unlikely to affect its stucture or function. If the cleavage site can be introduced where a Lys or His already exist, then the number of extraneous residues is reduced to one. This may be of more importance where potential immunogenicity is an issue.

The enormous benefit of using $^N$DKTH$^C$/Cu$^{2+}$ is in cost and convenience. Many proteases cannot be used for large or industrial scale processes due to the cost involved, and the problem of ensuring complete removal of the contaminating protease after cleavage. Assuming equal cleavage efficiencies, the cleavage of protein with CuCl$_2$ is approximately 1.2 x 10$^5$ fold cheaper than using Factor Xa protease. In addition, the CuCl$_2$ is neither animal derived or produced using animal products removing the risk of viral/prion contamination and can be removed simply from the protein sample by addition of EDTA, followed by dialysis. Cleaved material should be in the flow through after re-loading onto an affinity column, giving a simple method of separating cleaved and uncleaved material when the fusion partner is an affinity tail.

## REFERENCES

Adman ET (1991). Advances in Protein Chemistry <u>42,</u> 145-197.

Allen G, and Campbell R0 (1996). International Journal of Peptide and Protein Research. <u>48,</u> 265-273.

Brizzard BL et al (1994). Biotechniques <u>16,</u> 730-734.

Carter P, and Wells JA (1987). Science <u>237,</u> 394-399.

Casadaban MJ, et al (1983). Methods in Enzymology. <u>100,</u> 293-308.

Cooper B, et al (1985). Biochemical Journal. <u>228,</u> 615626.

Dawson RMC, et al (1989). Data for biochemical research. Oxford Science Publications, U.K.

Dean RT, *et al* (1989). Free Radical Research Communications. 7, 97-103.

Dougherty WG, *et al* (1988). Biochemical and mutational analysis of a plant virus polyprotein cleavage site. EMBO Journal 7, 1281-1287.

Dreher ML, *et al* (1991). Journal of Immunological Methods. 139, 197-205.

Easton CJ, *et al* (1997). Tetrahedron 53, 5609-5616.

Garrison W M (1968). Current Topics in Radiation Research, Volume 4.

di Guan C, *et* al (1988). Gene. 67: 21-30.

Hawkins C L, and Davies M J (1997). Biochimica et Biophysica Acta 1360, 84-96.

Hochuli E, *et al* (1988). Biotechnology. 6, 1321-1325.

Humphreys D P, *et al* (1997). JJournal of Immunological Methods. 209, 193-202.

Humphreys DP *et al* (1998). J. Immunol. 217, 1-10.

LaVallie E R, *et al* (1993). Journal of Biological Chemistry. 268, 2311-23317.

Marx G, and Chevion M (1985). Biochemical Journal. 236, 397-400.

McKenzie K R, *et al* (1 99 1). Journal of Immunology. 147, 312-319.

Nagai K, and Thogersen HC (1984). Nature. 309, 810-812.

Nilsson B, *et al* (1987). Protein Engineering. 1, 107113.

Ong E, *et al* (1989). Biotechnology. 7, 604-607.

Sadler P J, *et al* (1994). European Journal of Biochemistry. 220, 193200.

Schmidt TGM, and Skerra A (1994). Journal of Chromatography. 676, 337-345.

Smith DB, and Johnson KS (1988). Gene. 67, 31-40.

Smith M A, *et al* 1996). International Journal of peptide and Protein Research. 48, 48-55.

Stofko-Hahn RE, *et al* (1992). FEBS Letters. 302, 274-278.

Wada K N, *et al* (1991). Nucleic Acids Research. 19, 1981-1986.

Walker P A, *et al* (1994). Biotechnology. 12, 601-605.

Wolff S P, *et al* (1986). TIBS 11, 27-3 1.

**Claims**

1. A DNA coding for a fusion protein comprising two polypeptides joined by a cleavage site which does not naturally join the polypeptides and which is specifically cleavable by copper (II) ions, wherein said cleavage site is the tetrapeptide [N]DKTH[C], [N]DRSH[C], [N]EKSH[C] or [N]DKSH[C].

2. A vector comprising DNA according to claim 1.

**3.** A host cell transformed with a vector according to claim 2.

**4.** A process for the production of a desired protein in native form comprising:

1) expressing the desired protein as a fusion protein in a host cell according to claim 3 in which the desired protein is expressed as two polypeptides joined by a site specifically cleavable by copper (II) ions;
2) cleaving the fusion protein with copper (II) ions; and
3) recovering the desired cleaved protein in native form.

**5.** A process according to claim 4 wherein the desired protein is an antibody or a fragment thereof.

**Patentansprüche**

**1.** DNS, die ein Fusionsprotein kodiert, umfassend zwei Polypeptide, welche über eine Spaltstelle verbunden sind, welche die Polypeptide nicht natürlich verbindet und welche durch Kupfer(II)-Ionen spezifisch spaltbar ist, wobei die Spaltstelle das Tetrapeptid $^{N}$DKTH$^{C}$, $^{N}$DRSH$^{C}$, $^{N}$EKSH$^{C}$ oder $^{N}$DKSH$^{C}$ ist.

**2.** Vektor, umfassend die DNS nach Anspruch 1.

**3.** Wirtszelle, die mit einem Vektor nach Anspruch 2 transformiert ist.

**4.** Verfahren zur Herstellung eines gewünschten Proteins in nativer Form, umfassend:

1) das Exprimieren des gewünschten Proteins als ein Fusionsprotein in einer Wirtszelle nach Anspruch 3, in der das gewünschte Protein als zwei Polypeptide, verbunden über eine Stelle, welche durch Kupfer(II)-Ionen spezifisch spaltbar ist, exprimiert wird,
2) das Spalten des Fusionsproteins mit Kupfer(II)-Ionen und
3) das Rückgewinnen des gewünschten, gespaltenen Proteins in nativer Form.

**5.** Verfahren nach Anspruch 4, wobei das gewünschte Protein ein Antikörper oder ein Fragment davon ist.

**Revendications**

**1.** ADN codant pour une protéine de fusion comprenant deux polypeptides unis par un site de clivage qui n'unit pas naturellement les polypeptides et qui est spécifiquement clivable par des ions cuivre (II), dans lequel ledit site de clivage est le tétrapeptide $^{N}$DKTH$^{C}$, $^{N}$DRSH$^{C}$, $^{N}$EKSH$^{C}$ ou $^{N}$DKSH$^{C}$.

**2.** Vecteur comprenant un ADN selon la revendication 1.

**3.** Cellule hôte transformée avec un vecteur selon la revendication 2.

**4.** Procédé de production d'une protéine désirée sous forme native comprenant :

1) l'expression de la protéine désirée sous forme de protéine de fusion dans une cellule hôte selon la revendication 3 dans laquelle la protéine désirée est exprimée sous forme de deux polypeptides unis par un site spécifiquement clivable par des ions cuivre (II) ;
2) le clivage de la protéine de fusion avec des ions cuivre (II) ; et
3) la récupération de la protéine clivée désirée sous forme native.

**5.** Procédé selon la revendication 4 dans lequel la protéine désirée est un anticorps ou un de ses fragments.

## FIG. 1

## FIG. 2 Effect of pH on efficiency of cleavage by Cu(II)

## FIG. 3

## FIG. 4 Effect on extent of cleavage of Cu(II) concentration

## FIG. 5

## FIG. 6 Effect of duration of incubation on cleavage efficiency

## FIG. 7

### Effect of temperature of incubation on cleavage efficiency

% cleavage of FLAG tails

- ■— 59 deg. C
- ●— 61 deg. C
- ▲— 62 deg. C
- ✕— 64 deg. C

Time (hours)

## FIG. 10

pDPH76
6.58 Kb

Ptac
OmpA-vL
cK
Tet
OmpA-vH
CH1-hinge-FLAG
Spel
Scal
BamHI
HindIII
NotI
EcoRI
p15A ori
rrnB
laclq

## FIG. 8

## FIG. 9